(19) Europäisches Patentamt
European Patent Office
Office européen des brevets

(11) Veröffentlichungsnummer: **0 483 580 A2**

(12) **EUROPÄISCHE PATENTANMELDUNG**

(21) Anmeldenummer: **91117502.4**

(22) Anmeldetag: **14.10.91**

(51) Int. Cl.[5]: **C07D 207/08**, C07D 207/12, A61K 31/40, C07D 409/12, C07D 401/12

(30) Priorität: **02.11.90 DE 4034785**

(43) Veröffentlichungstag der Anmeldung: **06.05.92 Patentblatt 92/19**

(84) Benannte Vertragsstaaten: **AT BE CH DE DK ES FR GB GR IT LI LU NL SE**

(71) Anmelder: **MERCK PATENT GESELLSCHAFT MIT BESCHRÄNKTER HAFTUNG**
**Frankfurter Strasse 250**
**W-6100 Darmstadt(DE)**

(72) Erfinder: **Gottschlich, Rudolf, Dr.**
**Buchenweg 1**
**W-6107 Reinheim(DE)**
Erfinder: **Ackermann, Karl-August**
**Am Pfarrweiher 40**
**W-6105 Ober-Ramstadt(DE)**
Erfinder: **Prücher, Helmut**
**Königsbergerstrasse 9**
**W-6148 Heppenheim(DE)**
Erfinder: **Barber, Andrew, Dr.**
**Rudolf-Diesel-Strasse 15B**
**W-6108 Weiterstadt(DE)**
Erfinder: **Haase, Anton, Dr.**
**Ahornweg 5**
**W-6109 Mühltal 1(DE)**
Erfinder: **Greiner, Hartmut, Dr.**
**Dieburgerstrasse 218**
**W-6100 Darmstadt(DE)**
Erfinder: **Bartoszyk, Gerd**
**Heinrich-Fulda-Weg 22**
**W-6100 Darmstadt(DE)**

(54) **1-(2-Arylethyl)-Pyrrolidine.**

(57) Neue 1-(2-Arylethyl)-pyrrolidine der Formel I

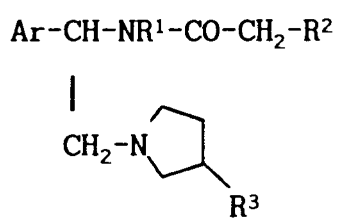

I

worin Ar, $R^1$, $R^2$ und $R^3$ die in Patentanspruch 1 angegebene Bedeutung haben, zeigen analgetische Eigenschaften.

EP 0 483 580 A2

Die Erfindung betrifft neue 1-(2-Arylethyl)-pyrrolidine der Formel I

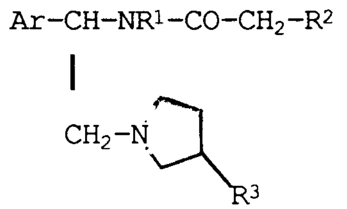

I

worin

Ar eine unsubstituierte oder eine einfach durch OH, $-O-CO-NH_2$, -O-CO-NHA, $-O-CO-NA_2$, $NH_2$, -NH-CHO, -NH-CO-A, $-NH-CO-NH_2$, -NH-CO-NHA, oder $NH-SO_2-A$ substituierte Phenylgruppe,

$R^1$ A,

$R^2$ eine unsubstituierte oder eine ein- oder zweifach durch A, Hal, $CF_3$, OH, OA, $-O-CO-NH_2$, -O-CO-NHA, $-O-CO-NA_2$, $NO_2$, $NH_2$, -NH-CHO, -NH-CO-A, $-NH-CO-NH_2$, -NH-CO-NHA, $-NH-SO_2A$, -CO-A, $-CONH_2$, -CONHA, $-CONA_2$, $-CH_2-CONH_2$ und/oder $-O-CH_2-CONH_2$ substituierte Phenyl-, Naphthyl-, Thienyl-, Benzothienyl- oder Pyridylgruppe,

$R^3$ OH oder $CH_2OH$,

A Alkyl mit 1-4 C-Atomen und

Hal F, Cl, Br oder I bedeuten,

sowie deren Salze.

In der DE-A1-39 35 371 sind Verbindungen einer Formel

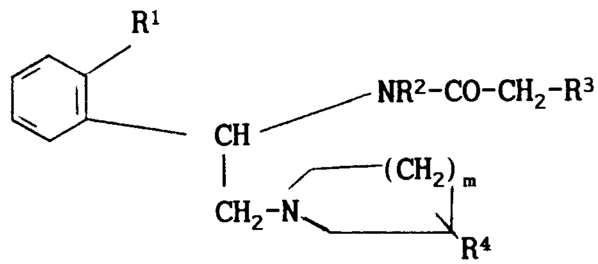

beschrieben, worin $R^1$ auch H, $R^2$ auch A, $R^3$ auch eine unsubstituierte oder eine in bestimmter Weise substituierte Phenyl-, Thienyl-, Naphthyl- oder Benzothienylgruppe, $R^4$ auch OH oder $CH_2OH$ und m auch 1 bedeuten kann. Jedoch sind dort weder Verbindungen der oben angegebenen Formel I noch irgendwelche Einzelverbindungen, die unter diese Formel I fallen, beschrieben. Verbindungen der Formel I sind daher im Hinblick auf die DE-A1-39 35 371 neu und besitzen demgegenüber den Charakter einer Auswahlerfindung.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre physiologisch unbedenklichen Salze wertvolle pharmakologische Eigenschaften besitzen. Sie zeigen eine analgetische Wirkung und antagonisieren insbesondere die entzündungsbedingte Hyperalgesie. So wirken die Verbindungen im "Writhing Test" an Mäusen oder Ratten (Methode vgl. Siegmund et al., Proc. Soc. Exp. Biol. 95, (1957), 729-731). Die analgetische Wirkung läßt sich ferner im "Tail-Flick-Test" an Mäusen oder Ratten nachweisen (Methodik vgl. d'Amour und Smith, J. Pharmacol. Exp. Ther. 72, (1941), 74-79), ferner im "Hot plate test" (vgl. Schmauss und Yaksh, J. Pharmacol. Exp. Ther. 228, (1984), 1-12 und die dort zitierte Literatur). Besonders starke Wirkungen sind an Ratten im Modell der Carrageenin-induzierten Hyperalgesie (vgl. Bartoszyk und Wild, Neuroscience Letters 101 (1989) 95) zu beobachten. Dabei zeigen die Verbindungen keine oder nur geringe Neigung zu physischer Abhängigkeit. Außerdem treten antiinflammatorische, antiasthmatische, diuretische, antikonvulsive und/oder antitussive Wirkungen auf, die ebenfalls nach hierfür geläufigen Methoden nachgewiesen werden können. Die Verbindungen eignen sich ferner zum Schutz vor und zur Behandlung von Hirnödemen und Unterversorgungszuständen des Zentralnervensystems, vor allem Hypoxie.

Die Verbindungen können daher als Arzneimittelwirkstoffe in der Human- und Veterinärmedizin verwendet werden. Ferner eignen sie sich als Zwischenprodukte zur Herstellung anderer Verbindungen mit wertvollen Eigenschaften.

Gegenstand der Erfindung sind Verbindungen der Formel I sowie ihre Salze.

Die Gruppe A steht für Alkyl mit 1, 2, 3 oder 4 C-Atomen, insbesondere für Methyl oder Ethyl, aber auch für Propyl, Isopropyl, Butyl, Isobutyl, sek.-Butyl, tert.-Butyl. Die Gruppe OA ist dementsprechend vorzugsweise Methoxy oder Ethoxy, ferner Propoxy, Isopropoxy, Butoxy, Isobutoxy, sek.-Butoxy oder tert.-Butoxy.

Dementsprechend haben die nachstehenden Gruppen die im folgenden genannten bevorzugten Bedeutungen:

| | |
|---|---|
| -O-CO-NHA: | N-Methyl-carbamoyl-oxy, N-Ethyl-carbamoyl-oxy; |
| -O-CO-$NA_2$: | N,N-Dimethyl-carbamoyl-oxy, N,N-Diethylcarbamoyl-oxy; |
| -NH-CO-A: | Acetamido, Propionamido; |
| -NH-CO-NHA: | N'-Methyl-ureido, N'-Ethyl-ureido; |
| -NH-$SO_2$-A: | Methylsulfonylamino, Ethylsulfonylamino; |
| -CO-A: | Acetyl, Propionyl; |
| -CO-NHA: | N-Methyl-carbamoyl, N-Ethyl-carbamoyl; |
| -$CONA_2$: | N'N-Dimethyl-carbamoyl, N,N-Diethyl-carbamoyl. |

Hal ist vorzugsweise Cl, ferner bevorzugt F, aber auch Br oder I.

Ar ist bevorzugt unsubstituiertes Phenyl, ferner bevorzugt o-, m- oder p-Aminophenyl, weiterhin bevorzugt o-, m- oder p-Hydroxyphenyl, o-, m- oder p-Formamidophenyl, o-, m- oder p-Acetamidophenyl, o-, m- oder p-Methylsulfonylaminophenyl, o-, m- oder p-Ureidophenyl, o-, m- oder p-N'-Methylureidophenyl. Unter den substituierten Phenylresten sind die in p-Stellung, aber auch die in m-Stellung bevorzugt.

$R^1$ ist bevorzugt Methyl.

Besonders bevorzugte Reste $R^2$ sind 3,4-Dichlorphenyl, o-, m-oder p-Nitrophenyl, o-, m- oder p-Aminophenyl, o-, m- oder p-Formamidophenyl, o-, m- oder p-Acetamidophenyl, o-, m- oder p-Ureidophenyl, o-, m- oder p-Carbamoylmethylphenyl, 1-Naphthyl und 4-Benzothienyl. Der Rest $R^2$ steht vorzugsweise aber auch für Phenyl, o-, m- oder p-Tolyl, o-, m- oder p-Ethylphenyl, o-, m- oder p-Methoxyphenyl, o-, m- oder p-Ethoxyphenyl, o-, m- oder p-Hydroxyphenyl, o-, m- oder p-Fluorphenyl, o-, m- oder p-Chlorphenyl, o-, m- oder p-Bromphenyl, o-, m- oder p-Trifluormethylphenyl, o-, m- oder p-Carbamoyloxy-phenyl, o-, m- oder p-N-Methylcarbamoyloxyphenyl, o-, m- oder p-N,N-Dimethylcarbamoyloxy-phenyl, o-, m- oder p-N'-Methylureido-phenyl, o-, m- oder p-Methylsulfonylamino-phenyl, o-, m- oder p-Acetyl-phenyl, o-, m- oder p-Carbamoylphenyl, o-, m- oder p-N-Methylcarbamoyl-phenyl, o-, m- oder p-N,N-Dimethylcarbamoyl-phenyl, o-, m- oder p-Carbamoylmethyl-phenyl, o-, m- oder p-Carbamoylmethoxy-phenyl, 2,3-, 2,4-, 2,5-, 2,6-, 3,4- oder 3,5-Dimethylphenyl, 2,3-, 2,4-, 2,5-, 2,6-, 3,4- oder 3,5-Dimethoxyphenyl, 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2-Amino-3-chlorphenyl, 2-Amino-4-chlorphenyl, 2-Amino-5-chlorphenyl, 2-Amino-6-chlorphenyl, 3-Amino-2-chlorphenyl, 3-Amino-4-chlorphenyl, 3-Amino-5-chlorphenyl, 3-Amino-6-chlorphenyl, 4-Amino-2-chlorphenyl, 4-Amino-3-chlorphenyl, 2-Amino-3-, -4-, -5-, oder -6-bromphenyl, 3-Amino-2-, -4-, -5- oder -6-bromphenyl, 4-Amino-2- oder -3-bromphenyl, 2-Chlor-4-hydroxyphenyl, 3-Chlor-4 hydroxyphenyl, 2-Hydroxy-4-chlorphenyl, 3-Hydroxy-4-chlorphenyl, 3-Chlor-4-carboxymethoxyphenyl, 3-Chlor-4-methoxycarbonylmethoxyphenyl, 3-Chlor-4-ethoxycarbonylmethoxyphenyl, 2-Naphthyl, 2-, 3-, 4-, 5-, 6-, 7- oder 8-Chlor-1-naphthyl, 2- oder 3-Thienyl, 3-, 4-, oder 5-Chlor-2-thienyl, 3-, 4-, oder 5-Brom-2-thienyl, 2-, 4-, oder 5-Chlor-3-thienyl, 2-, 4-, oder 5-Brom-3-thienyl, 3,4-, 3,5- oder 4,5-Dichlor-2-thienyl, 5-Amino-2-thienyl, 5-Formamido-2-thienyl, 5-Acetamido-2-thienyl, 5-Methylsulfonylamino-2-thienyl, 5-Ureido-2-thienyl, 5-N'-Methylureido-2-thienyl, 2-, 3-, 5-, 6- oder 7-Benzothienyl, 2-, 3- oder 4-Pyridyl, 3-Amino-4-pyridyl, 3-Formamido-4-pyridyl, 3-Acetamido-4-pyridyl, 3-Methylsulfonylamino-4-pyridyl, 3-Ureido-4-pyridyl, 3-N'-Methylureido-4-pyridyl, 3-Amino-4-methyl-2-pyridyl, 3-Formamido-4-methyl-2-pyridyl, 3-Acetamido-4-methyl-2-pyridyl, 4-Methyl-3-methylsulfonylamino-2-pyridyl, 4-Methyl-3-ureido-2-pyridyl, 4-Methyl-3-N'-methylureido-2-pyridyl, 5-Amino-4-methyl-3-pyridyl, 5-Formamido-4-methyl-2-pyridyl, 5-Acetamido-4-methyl-2-pyridyl, 4-Methyl-5-methylsulfonylamino-2-pyridyl, 4-Methyl-5-ureido-2-pyridyl, 4-Methyl-5-N'-methylureido-2-pyridyl.

$R^3$ ist vorzugsweise OH, aber auch $CH_2OH$.

Gegenstand der Erfindung sind im einzelnen Verbindungen der Formeln Ia und Ib, worin die nicht näher bezeichneten Reste die bei Formel I angegebenen Bedeutungen haben, worin jedoch in Ia $R^3$ OH und in Ib $R^3$ $CH_2OH$ bedeutet.

Gegenstand der Erfindung sind insbesondere diejenigen Verbindungen der Formeln I, Ia und Ib, in denen mindestens einer der genannten Reste eine der vorstehend angegebenen bevorzugten Bedeutungen hat. Einige bevorzugte Gruppen von Verbindungen können durch die Teilformeln I' sowie Ia' und Ib' ausgedrückt werden, die den Formeln I (sowie Ia und Ib) entsprechen und worin die nicht näher bezeichneten Reste die bei der Formel I angegebenen Bedeutungen haben, worin jedoch $R^2$ Phenyl, Tolyl, Methoxyphenyl, Hydroxyphenyl, Fluorphenyl, Chlorphenyl, Bromphenyl, Dichlorphenyl, Aminochlorphenyl, Aminobromphenyl, Chlorhydroxyphenyl, Nitrophenyl, Aminophenyl, Ureidophenyl, Carbamoylmethylphenyl, Carbamoylphenyl, Carbamoylmethoxyphenyl, Formamidophenyl, Acetamidophenyl, Methylsulfonamidophenyl, N'-Methylureidophenyl, N-Methylcarbamoylphenyl, Naphthyl, Thienyl, Benzothienyl oder Pyridyl bedeutet.

Ferner sind bevorzugt Verbindungen der Formeln I'' sowie Ia'' und Ib'', die den Formeln I sowie Ia und Ib entsprechen, worin jedoch

$R^2$ Dichlorphenyl, Aminochlorphenyl, Aminobromphenyl, Nitrophenyl, Aminophenyl, Acetamidophenyl oder Ureidophenyl

bedeutet.

Weiterhin sind bevorzugt Verbindungen der Formeln I''' sowie Ia''' und Ib''', die den Formeln I sowie Ia und Ib entsprechen, worin jedoch

$R^2$ 3,4-Dichlorphenyl, o- oder p-Nitrophenyl, o- oder p-Aminophenyl, o- oder p-Acetamidophenyl oder o- oder p-Ureidophenyl

bedeutet.

Insbesondere sind diejenigen Verbindungen der Formeln I, Ia, Ib, I', Ia', Ib', I'', Ia'', Ib'', I''', Ia''' und Ib''' bevorzugt, in denen Ar eine unsubstituierte Phenylgruppe bedeutet.

Weiterhin sind diejenigen unter allen genannten Verbindungen bevorzugt, in denen $R^1$ Methyl bedeutet.

Gegenstand der Erfindung ist ferner ein Verfahren zur Herstellung von 1-(2-Phenylethyl)-pyrrolidinen der Formel I gemäß Anspruch 1 sowie ihrer Salze, dadurch gekennzeichnet, daß man eine Verbindung der Formel II

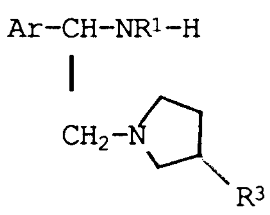

II

worin Ar, $R^1$ und $R^3$ die bei Formel I angegebene Bedeutung haben, mit einer Verbindung der Formel III

$HOOC\text{-}CH_2\text{-}R^2$ III

worin $R^2$ die bei Formel I angegebene Bedeutung hat, oder einem ihrer funktionellen Derivate umsetzt oder daß man eine Verbindung, die sonst der Formel I entspricht, aber an Stelle eines oder mehrerer H-Atome eine oder mehrere reduzierbare oder hydrogenolytisch abspaltbare Gruppen und/oder C-C- und/oder C-N-Bindungen enthält, mit einem reduzierenden Mittel behandelt,

oder daß man zur Herstellung einer Verbindung der Formel I, die eine Säureamidgruppe enthält, eine entsprechende Carbonsäure oder einen ihrer Ester mit Ammoniak oder mit einem Amin der Formeln $A\text{-}NH_2$ oder $A_2NH$ umsetzt,

und/oder daß man in einer Verbindung der Formel I einen oder mehrere der Reste Ar und/oder $R^2$ in einen oder mehrere andere Reste Ar und/oder $R^2$ umwandelt,

und/oder daß man eine Base der Formel I durch Behandeln mit einer Säure in eines ihrer Salze überführt.

Die Verbindungen der Formel I werden in der Regel nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe sind in der Regel bekannt, oder sie können in Analogie zu bekannten Stoffen nach an sich bekannten Verfahren hergestellt werden. Sie können gewünschtenfalls auch in situ gebildet werden, derart, daß man sie aus dem Reaktionsgemisch nicht isoliert, sondern sofort weiter zu den Verbindungen der Formel I umsetzt. Andererseits ist es möglich, die Reaktion stufenweise durchzuführen, wobei man weitere Zwischenprodukte isolieren kann.

Die einzelnen Verfahrensvarianten werden im folgenden näher erläutert.

Die Verbindungen der Formel I sind bevorzugt herstellbar durch Reaktion der Verbindungen der Formel II mit Carbonsäuren der Formel III oder ihren funktionellen Derivaten. Als funktionelle Derivate der Verbindungen der Formel III eignen sich insbesondere die entsprechenden Ester, vor allem die Methyl- oder Ethylester, die Halogenide, Anhydride, Azide oder Nitrile; die Chloride sind bevorzugt.

Verbindungen der Formel II sind beispielsweise erhältlich durch Umsetzung von α-Alkanoylamino-phenylessigsäuren (worin die Alkanoylgruppe 1-4 C-Atome hat; z.B. α-Formamido-phenylessigsäure) mit 3-$R^3$-pyrrolidinen (3-Hydroxypyrrolidin oder 3-Hydroxymethylpyrrolidin) zu den entsprechenden 3-$R^3$-pyrrolididen und anschließende gleichzeitige Reduktion der beiden Amidgruppen mit $LiAlH_4$.

Typische Verbindungen der Formel II sind z.B. 1-(2-Methyl-, 1-(2-Ethyl-, 1-(2-Propyl-, 1-(2-Isopropyl- oder 1-(2-Butyl-amino-2-phenyl-ethyl)-3-hydroxy-pyrrolidin, 1-(2-Methyl-, 1-(2-Ethyl-, 1-(2-Propyl-, (1-(2-Isopropyl- oder (1-(2-Butyl-amino-2-phenyl-ethyl)-3-hydroxymethyl-pyrrolidin.

Typische Verbindungen der Formel III sind z.B. Phenylacetylchlorid, -bromid und -azid, Phenylessigsäure-methyl- und -ethylester, (Phenylessigsäure)-anhydrid, Phenylacetonitril sowie die entsprechenden Derivate der 3,4-Dichlorphenylessigsäure (z.B. 3,4-Dichlorphenylacetylchlorid), der 1-Naphthylessigsäure (z.B. 1-Naphthylacetylchlorid), der 4-Benzothienylessigsäure (z.B. 4-Benzothienylacetylchlorid) und der o-, m- oder p-Nitrophenylessigsäure (z.B. o-, m- oder p-Nitrophenylacetylchlorid).

Die Umsetzung von II mit III bzw. III-Derivaten gelingt zweckmäßig in Anwesenheit oder Abwesenheit eines inerten organischen Lösungsmittels, z.B. eines halogenierten Kohlenwasserstoffs wie Dichlormethan, Chloroform oder Trichlorethen, eines Alkohols wie Methanol, Ethanol oder Butanol, eines Ethers wie Tetrahydrofuran (THF) oder Dioxan, eines Amids wie Dimethylformamid (DMF), eines Sulfoxids wie Dimethylsulfoxid (DMSO) und/oder in Gegenwart oder Abwesenheit eines Kondensationsmittels, z.B. einer Base, bei Temperaturen zwischen -20 und 200°, vorzugsweise 0 und 100°. Als Basen eignen sich z.B. Alkalimetallhydroxide wie NaOH oder KOH, Alkalimetallcarbonate wie $Na_2CO_3$ oder $K_2CO_3$, tertiäre Amine wie Triethylamin oder Pyridin. Als Lösungsmittel ist Dichlormethan, als Base Triethylamin besonders bevorzugt.

Als Ausgangsstoffe für die Herstellung von Verbindungen der Formel I durch Reduktion entsprechender Verbindungen, die an Stelle von H-Atomen eine oder mehrere zusätzliche reduzierbare oder hydrogenolytisch abspaltbare Gruppen und/oder C-C-Bindungen und/oder C-N-Bindungen enthalten, eignen sich insbesondere Verbindungen der Formel IV

$$Ar'-\underset{\displaystyle R^5-R^6}{\underset{|}{CH}}-NR^{1'}-CO-CH_2-R^4 \qquad IV$$

worin

Ar'

(a) Ar,

(b) einen sonst Ar entsprechenden Rest, der aber an Stelle des H-Atoms einer OH-Gruppe oder einer $NH_2$-Gruppe einen hydrogenolytisch abspaltbaren Rest oder an Stelle einer $NH_2$-Gruppe eine $NO_2$-Gruppe enthält,

$R^{1'}$

(a) $R^1$,

(b) eine Oxo-alkylgruppe mit 1-4 C-Atomen,

$R^4$

(a) $R^2$,

(b) einen sonst R entsprechenden Rest, der aber an Stelle des H-Atoms einer OH-Gruppe oder einer $NH_2$-Gruppe einen hydrogenolytisch abspaltbaren Rest enthält,

$R^5$

(a) $-CH_2-$,

(b) -CO- und

$R^6$

(a) 3-$R^3$-pyrrolidino,

(b) 3-Oxo-pyrrolidino, 3-Formylpyrrolidino oder 2-, 4- oder 5-Oxo-3-$R^3$-pyrrolidino

bedeuten; dabei muß IV jedoch von I verschieden sein, d.h. die Reste Ar', $R^{1'}$, $R^4$, $R^5$ und $R^6$ können nicht gleichzeitig die jeweils unter (a) angegebenen Bedeutungen haben.

Der Rest $R^4$ kann vorzugsweise o-, m- oder p-Benzyloxyphenylacetyl bedeuten.

Als reduzierendes Mittel eignet sich vorzugsweise Wasserstoff in Gegenwart eines Katalysators, insbesondere eines Edelmetall-, Nickel- oder Kobaltkatalysators. Als Edelmetalle kommen in erster Linie Platin und Palladium in Betracht, die auf Trägern (z.B. auf Kohle, Calciumcarbonat oder Strontiumcarbonat), als Oxide (z.B. Platinoxid) oder in feinteiliger Form vorliegen können. Nickel- und Kobaltkatalysatoren werden zweckmäßig als Raney-Metalle eingesetzt. Man hydriert zweckmäßig bei Drucken zwischen etwa 1 und etwa 200 bar und bei Temperaturen zwischen etwa -80 und +150°, vorzugsweise zwischen 20 und 100°, in Gegenwart eines inerten Lösungsmittels, z.B. eines Alkohols wie Methanol, Ethanol oder Isopropanol, einer Carbonsäure wie Essigsäure, eines Esters wie Ethylacetat, eines Ethers wie THF oder Dioxan.

Zur Herstellung einer Verbindung der Formel I, die eine Säureamidgruppe, also eine (oder mehrere) der Gruppen $-CONH_2$, -CONHA oder $-CONA_2$, enthält, kann man eine entsprechende Carbonsäure oder einen entsprechenden Ester, vorzugsweise einen niederen Alkylester, der an Stelle der Säureamidgruppe die Gruppe COOA enthält, mit Ammoniak oder einem Amin der Formeln $ANH_2$ oder $A_2NH$ umsetzen. Carbonsäuren setzt man zweckmäßig um in Gegenwart eines Dehydratisierungsmittels wie Dicyclohexylcarbodiimid oder Carbonyldiimidazol in einem inerten Lösungsmittel wie DMF bei 15-40°.

Falls erwünscht, kann man in einer Verbindung der Formel I einen oder mehrere der Reste Ar und/oder $R^2$ gegen einen oder mehrere andere Reste Ar und/oder $R^2$ austauschen.

So kann man Ethergruppen (z.B. OA-Gruppen) unter Bildung von OH-Gruppen spalten, z.B. durch Behandeln mit Dimethylsulfid-Bortribromid-Komplex, z.B. in Toluol, THF oder DMSO, oder durch Verschmelzen mit Pyridin- oder Anilinhydrohalogeniden, vorzugsweise Pyridinhydrochlorid, bei etwa 150-250°, oder durch Behandeln mit Diisobutylaluminiumhydrid in Toluol bei etwa 0-110°.

Weiterhin kann man OH-Gruppen verethern, z.B. indem man zunächst die entsprechenden Alkalimetall- (z.B. Na- oder K-)alkoholate, -phenolate oder Salze herstellt und diese mit entsprechenden Halogenverbindungen umsetzt, z.B. mit Alkylhalogeniden wie Methylchlorid, -bromid oder -iodid, Chloroder Bromacetamid, zweckmäßig in Gegenwart eines der oben angegebenen Lösungsmittel bei Temperaturen zwischen 0 und 100°.

Nitrogruppen können zu Aminogruppen reduziert werden, zweckmäßig durch katalytische Hydrierung unter den oben angegebenen Bedingungen, z.B. mit Raney-Ni im Methanol oder Ethanol bei 15-40° und Normaldruck.

Aminogruppen können acyliert werden, z.B. mit Säurechloriden wie Acetyl-, Methansulfonyl-, Oxalsäure- oder Bernsteinsäurehalbester-chlorid, zweckmäßig in inerten Lösungsmitteln wie Dichlormethan bei 15-40°. Eine Formylierung von Aminogruppen gelingt auch durch mehrstündige Reaktion mit überschüssiger Ameisensäure bei 80-100°. Umsetzung von primären Aminoverbindungen mit Cyanaten, z.B. mit KCNO in Wasser bei 15-40°, gibt die entsprechenden Ureidoverbindungen; mit Alkylisocyanaten, z.B. in inerten Lösungsmitteln wie THF bei 15-40°, erhält man entsprechend N'-Alkyl-ureidoverbindungen.

Eine Base der Formel I kann mit einer Säure in das zugehörige Säureadditionssalz übergeführt werden. Für diese Umsetzung kommen Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalinmono- und disulfonsäuren, Laurylschwefelsäure. Salze mit physiologisch nicht unbedenklichen Säuren, z.B. Pikrate, können zur Aufreinigung der Verbindungen der Formel I verwendet werden.

Die freien Basen der Formel I können, falls gewünscht, aus ihren Salzen durch Behandlung mit starken Basen wie Natriumoder Kaliumhydroxid, Natrium- oder Kaliumcarbonat in Freiheit gesetzt werden.

Die Verbindungen der Formel I enthalten mindestens zwei chirale Zentren und können daher in racemischer oder in optisch-aktiver Form vorliegen. Erhaltene Racemate können nach an sich bekannten Methoden mechanisch oder chemisch in die Enantiomeren getrennt werden. Vorzugsweise werden aus dem

racemischen Gemisch durch Umsetzung mit einem optisch-aktiven Trennmittel Diastereomere gebildet. Als Trennmittel eignen sich z.B. optisch aktive Säuren, wie die D- und L-Formen von Weinsäure, Diacetylweinsäure, Dibenzoylweinsäure, Mandelsäure, Äpfelsäure, Milchsäure oder die verschiedenen optischaktiven Camphersulfonsäuren wie β-Camphersulfonsäure.

Vorteilhaft ist auch eine Enantiomerentrennung mit Hilfe einer mit einem optisch aktiven Trennmittel (z.B. Dinitrobenzoyl-phenyl-glycin) gefüllten Säule; als Laufmittel eignet sich z.B. ein Gemisch Hexan/Isopropanol/Acetonitril, z.B. im Volumenverhältnis 82:15:3.

Natürlich ist es auch möglich, optisch-aktive Verbindungen der Formel I nach den oben beschriebenen Methoden zu erhalten, indem man Ausgangsstoffe (z.B. solche der Formel II) verwendet, die bereits optisch-aktiv sind.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können bei der Bekämpfung von Krankheiten, insbesondere von Schmerzzuständen verwendet werden.

Dabei werden die erfindungsgemäßen Substanzen in der Regel in Analogie zu bekannten Analgetika verabreicht, vorzugsweise in Dosierungen zwischen etwa 1 und 500 mg, insbesondere zwischen 5 und 100 mg pro Dosierungseinheit. Die tägliche Dosierung liegt vorzugsweise zwischen etwa 0,02 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser oder verdünnte Natronlauge hinzu, extrahiert mit Dichlormethan, trennt ab, trocknet die organische Phase mit Natriumsulfat, filtriert, dampft ein und reinigt durch Chromatographie an Kieselgel und/oder durch Kristallisation.

HCl' = Hydrochlorid. Rf = Rf-Wert auf Dünnschicht-Kieselgel 60 $F_{254}$ (E. Merck, Art.-Nr. 5715), $CH_2Cl_2/CH_3OH$ 9:1 mit Zusatz von 0,5 % Triethylamin.

$[\alpha] = [\alpha]_D^{20}$, c = 1 in Methanol.

Beispiel 1

Eine Lösung von 22 g 1-(2-Methylamino-2-phenyl-ethyl)-3-hydroxy-pyrrolidin [Di-HCl', F. 201°; erhältlich durch Umsetzung von α-Formamido-phenylessigsäure mit 3-Hydroxypyrrolidin zu α-Formamido-phenylessigsäure-(3-hydroxypyrrolidid) (Öl) und Reduktion mit $LiAlH_4$] in 250 ml Dichlormethan wird mit 30 ml Triethylamin versetzt. Anschließend tropft man unter Rühren eine Lösung von 22,4 g 3,4-Dichlorphenylacetylchlorid in 200 ml Dichlormethan hinzu, rührt noch 2 Std. bei 20° und erhält nach üblicher Aufarbeitung 1-[2-(N-3,4-Dichlorphenylacetyl-N-methyl-amino)-2-phenylethyl]-3-hydroxy-pyrrolidin. Rf 0,37. HCl', F. 201°.

Beispiel 2

Analog Beispiel 1 erhält man aus 1-(2-Methylamino-2-phenylethyl)-3-hydroxymethyl-pyrrolidin [erhältlich durch Reduktion von α-Formamido-phenylessigsäure-(3-hydroxymethyl-pyrrolidid) mit $LiAlH_4$] und 3,4-Dichlorphenylacetylchlorid das 1-[2-(N-3,4-Dichlorphenylacetyl-N-methyl-amino)-2-phenylethyl]-3-hydroxymethyl-pyrrolidin, Rf 0,36. HCl', F. 225°.

Beispiel 3

Analog Beispiel 1 erhält man mit p-Nitrophenylacetylchlorid das 1-[2-(N-Methyl-N-p-nitrophenylacetyl-amino)-2-phenylethyl]-3-hydroxy-pyrrolidin, Rf 0,34.

Aus α-Formamido-phenylessigsäure-(3R-hydroxy-pyrrolidid) erhält man über 1-(2-Methylamino-2-phenyl-ethyl)-3R-hydroxypyrrolidinanalog das 1-[2-(N-Methyl-N-p-nitrophenylacetyl-amino)-2-phenyl-ethyl]-3R-hydroxy-pyrrolidin (Rf 0,34; [α] -2,2°. HCl', F. 235°) sowie das 1-[2-(N-3,4-Dichlorphenylacetyl-N-methyl-amino)-2-phenyl-ethyl]-3R-hydroxypyrrolidin, Rf 0,37.

Aus αS-Formamido-phenylessigsäure-(3-hydroxy-pyrrolidid) erhält man über 1-(2S-Methylamino-2-phenyl-ethyl)-3-hydroxypyrrolidinanalog das 1-[2S-(N-Methyl-N-p-nitrophenylacetyl-amino)-2-phenyl-ethyl]-3-hydroxy-pyrrolidin, Rf 0,34.

Aus αS-Formamido-phenylessigsäure-(3S-hydroxy-pyrrolidid erhält man über 1-(2S-Methylamino-2-phenyl-ethyl)-3S-hydroxypyrrolidin analog das 1-[2S-(N-Methyl-N-p-nitrophenylacetylamino)-2-phenyl-ethyl]-3S-hydroxy-pyrrolidin, Rf 0,34.

Analog erhält man mit o- bzw. m-Nitrophenylacetylchlorid:

1-[2-(N-Methyl-N-o-nitrophenylacetyl-amino)-2-phenylethyl]-3-hydroxy-pyrrolidin
1-[2-(N-Methyl-N-o-nitrophenylacetyl-amino)-2-phenylethyl]-3R-hydroxy-pyrrolidin
1-[2-(N-Methyl-N-o-nitrophenylacetyl-amino)-2-phenylethyl]-3S-hydroxy-pyrrolidin, Rf 0,35
1-[2S-(N-Methyl-N-o-nitrophenylacetyl-amino)-2-phenylethyl]-3-hydroxy-pyrrolidin, Rf 0,35
1-[2S-(N-Methyl-N-o-nitrophenylacetyl-amino)-2-phenylethyl]-3S-hydroxy-pyrrolidin, Rf 0,35
1-[2S-(N-Methyl-N-o-nitrophenylacetyl-amino)-2-phenylethyl]-3R-hydroxy-pyrrolidin, Rf 0,35
1-[2-(N-Methyl-N-m-nitrophenylacetyl-amino)-2-phenylethyl]-3-hydroxy-pyrrolidin
1-[2-(N-Methyl-N-m-nitrophenylacetyl-amino)-2-phenylethyl]-3R-hydroxy-pyrrolidin
1-[2-(N-Methyl-N-m-nitrophenylacetyl-amino)-2-phenylethyl]-3S-hydroxy-pyrrolidin
1-[2S-(N-Methyl-N-m-nitrophenylacetyl-amino)-2-phenylethyl]-3-hydroxy-pyrrolidin, Rf 0,36.

Beispiel 4

Analog Beispiel 1 erhält man

mit o-, m- oder p-Methylphenylacetylchlorid:

1-[2-(N-o-Methylphenylacetyl-N-methyl-amino)-2-phenylethyl]-3-hydroxy-pyrrolidin
1-[2-(N-m-Methylphenylacetyl-N-methyl-amino)-2-phenylethyl]-3-hydroxy-pyrrolidin
1-[2-(N-p-Methylphenylacetyl-N-methyl-amino)-2-phenylethyl]-3-hydroxy-pyrrolidin;

mit o-, m- oder p-Fluorphenylacetylchlorid:

1-[2-(N-o-Fluorphenylacetyl-N-methyl-amino)-2-phenylethyl]-3-hydroxy-pyrrolidin
1-[2-(N-m-Fluorphenylacetyl-N-methyl-amino)-2-phenylethyl]-3-hydroxy-pyrrolidin
1-[2-(N-p-Fluorphenylacetyl-N-methyl-amino)-2-phenylethyl]-3-hydroxy-pyrrolidin;

mit o-, m- oder p-Chlorphenylacetylchlorid:

1-[2-(N-o-Chlorphenylacetyl-N-methyl-amino)-2-phenylethyl]-3-hydroxy-pyrrolidin
1-[2-(N-m-Chlorphenylacetyl-N-methyl-amino)-2-phenylethyl]-3-hydroxy-pyrrolidin
1-[2-(N-p-Chlorphenylacetyl-N-methyl-amino)-2-phenylethyl]-3-hydroxy-pyrrolidin;

mit o-, m- oder p-Bromphenylacetylchlorid:

1-[2-(N-o-Bromphenylacetyl-N-methyl-amino)-2-phenyl-ethyl]-3-hydroxy-pyrrolidin
1-[2-(N-m-Bromphenylacetyl-N-methyl-amino)-2-phenyl-ethyl]-3-hydroxy-pyrrolidin
1-[2-(N-p-Bromphenylacetyl-N-methyl-amino)-2-phenyl-ethyl]-3-hydroxy-pyrrolidin;

mit o-, m- oder p-Trifluormethyl-phenylacetylchlorid:

1-[2-(N-Methyl-N-o-trifluorphenylacetyl-amino)-2-phenylethyl]-3-hydroxy-pyrrolidin
1-[2-(N-Methyl-N-m-trifluorphenylacetyl-amino)-2-phenylethyl]-3-hydroxy-pyrrolidin
1-[2-(N-Methyl-N-p-trifluorphenylacetyl-amino)-2-phenylethyl]-3-hydroxy-pyrrolidin;

mit o-, m- oder p-Methoxyphenylacetylchlorid:

1-[2-(N-o-Methoxyphenylacetyl-N-methyl-amino)-2-phenylethyl]-3-hydroxy-pyrrolidin

1-[2-(N-m-Methoxyphenylacetyl-N-methyl-amino)-2-phenylethyl]-3-hydroxy-pyrrolidin
1-[2-(N-p-Methoxyphenylacetyl-N-methyl-amino)-2-phenylethyl]-3-hydroxy-pyrrolidin;
mit o-, m- oder p-Acetylphenylacetylchlorid:
1-[2-(N-o-Acetylphenylacetyl-N-methyl-amino)-2-phenylethyl]-3-hydroxy-pyrrolidin
1-[2-(N-m-Acetylphenylacetyl-N-methyl-amino)-2-phenylethyl]-3-hydroxy-pyrrolidin
1-[2-(N-p-Acetylphenylacetyl-N-methyl-amino)-2-phenylethyl]-3-hydroxy-pyrrolidin;
mit o-, m- oder p-Carbamoylphenylacetylchlorid:
1-[2-(N-o-Carbamoylphenylacetyl-N-methyl-amino)-2-phenylethyl]-3-hydroxy-pyrrolidin
1-[2-(N-m-Carbamoylphenylacetyl-N-methyl-amino)-2-phenylethyl]-3-hydroxy-pyrrolidin
1-[2S-(N-m-Carbamoylphenylacetyl-N-methyl-amino)-2-phenylethyl]-3S-hydroxy-pyrrolidin, Rf 0,15
1-[2-(N-p-Carbamoylphenylacetyl-N-methyl-amino)-2-phenylethyl]-3-hydroxy-pyrrolidin
1-[2S-(N-p-Carbamoylphenylacetyl-N-methyl-amino)-2-phenylethyl]-3S-hydroxy-pyrrolidin, Rf 0,12;
mit o-, m- oder p-(N-Methylcarbamoyl)-phenylacetylchlorid:
1-[2-(N-Methyl-N-o-(N-methylcarbamoyl)-phenylacetyl-amino)-2-phenyl-ethyl]-3-hydroxy-pyrrolidin
1-[2-(N-Methyl-N-m-(N-methylcarbamoyl)-phenylacetyl-amino)-2-phenyl-ethyl]-3-hydroxy-pyrrolidin
1-[2-(N-Methyl-N-p-(N-methylcarbamoyl)-phenylacetyl-amino)-2-phenyl-ethyl]-3-hydroxy-pyrrolidin;
mit o-, m- oder p-(N,N-Dimethylcarbamoyl)-phenylacetylchlorid:
1-[2-(N-o-(N,N-Dimethylcarbamoyl)-phenylacetyl-N-methylamino)-2-phenyl-ethyl]-3-hydroxy-pyrrolidin
1-[2-(N-p-(N,N-Dimethylcarbamoyl)-phenylacetyl-N-methylamino)-2-phenyl-ethyl]-3-hydroxy-pyrrolidin
1-[2-(N-m-(N,N-Dimethylcarbamoyl)-phenylacetyl-N-methylamino)-2-phenyl-ethyl]-3-hydroxy-pyrrolidin;
mit o-, m- oder p-Carbamoylmethyl-phenylacetylchlorid:
1-[2-(N-o-Carbamoylmethyl-phenylacetyl-N-methyl-amino)-2-phenyl-ethyl]-3-hydroxy-pyrrolidin
1-[2-(N-m-Carbamoylmethyl-phenylacetyl-N-methyl-amino)-2-phenyl-ethyl]-3-hydroxy-pyrrolidin
1-[2-(N-p-Carbamoylmethyl-phenylacetyl-N-methyl-amino)-2-phenyl-ethyl]-3-hydroxy-pyrrolidin
1-[2S-(N-p-Carbamoylmethyl-phenylacetyl-N-methyl-amino)-2-phenyl-ethyl]-3S-hydroxy-pyrrolidin, Rf 0,18;
mit o-, m- oder p-Carbamoylmethoxy-phenylacetylchlorid:
1-[2-(N-o-Carbamoylmethoxy-phenylacetyl-N-methyl-amino)-2-phenyl-ethyl]-3-hydroxy-pyrrolidin
1-[2-(N-m-Carbamoylmethoxy-phenylacetyl-N-methyl-amino)-2-phenyl-ethyl]-3-hydroxy-pyrrolidin
1-[2-(N-p-Carbamoylmethoxy-phenylacetyl-N-methyl-amino)-2-phenyl-ethyl]-3-hydroxy-pyrrolidin;
mit Phenylacetylchlorid:
1-[2-(N-Methyl-N-phenylacetyl-amino)-2-phenyl-ethyl]-3-hydroxy-pyrrolidin;
mit 1- oder 2-Naphthylacetylchlorid:
1-[2-(N-Methyl-N-(1-naphthylacetyl-amino)-2-phenyl-ethyl]-3-hydroxy-pyrrolidin
1-[2-(N-Methyl-N-(2-naphthylacetyl-amino)-2-phenyl-ethyl]-3-hydroxy-pyrrolidin;
mit 2- oder 3-Thienylacetylchlorid:
1-[2-(N-Methyl-N-(2-thienylacetyl-amino)-2-phenyl-ethyl]-3-hydroxy-pyrrolidin
1-[2-(N-Methyl-N-(3-thienylacetyl-amino)-2-phenyl-ethyl]-3-hydroxy-pyrrolidin;
mit 4-Benzothienyl-acetylchlorid:
1-[2-(N-(4-Benzothienylacetyl-N-methyl-amino)-2-phenyl-ethyl]-3-hydroxy-pyrrolidin;
mit 2-, 3- oder 4-Pyridylacetylchlorid:
1-[2-(N-Methyl-N-(2-pyridyl)-acetyl-amino)-2-phenyl-ethyl]-3-hydroxy-pyrrolidin
1-[2-(N-Methyl-N-(3-pyridyl)-acetyl-amino)-2-phenyl-ethyl]-3-hydroxy-pyrrolidin
1-[2-(N-Methyl-N-(4-pyridyl)-acetyl-amino)-2-phenyl-ethyl]-3-hydroxy-pyrrolidin;
mit 5-Nitro-2-thienyl-acetylchlorid:
1-[2-(N-Methyl-N-(5-nitro-2-thienyl-acetyl)-amino)-2-phenyl-ethyl]-3-hydroxy-pyrrolidin;
mit 3-, 4-, 5- oder 6-Chlor-2-nitrophenylacetylchlorid:
1-[2-(N-3-Chlor-2-nitrophenylacetyl-N-methyl-amino)-2-phenylethyl]-3-hydroxy-pyrrolidin
1-[2-(N-4-Chlor-2-nitrophenylacetyl-N-methyl-amino)-2-phenylethyl]-3-hydroxy-pyrrolidin
1-[2-(N-5-Chlor-2-nitrophenylacetyl-N-methyl-amino)-2-phenylethyl]-3-hydroxy-pyrrolidin
1-[2-(N-6-Chlor-2-nitrophenylacetyl-N-methyl-amino)-2-phenylethyl]-3-hydroxy-pyrrolidin;
mit 2-, 4-, 5- oder 6-Chlor-3-nitrophenylacetylchlorid:
1-[2-(N-2-Chlor-3-nitrophenylacetyl-N-methyl-amino)-2-phenylethyl]-3-hydroxy-pyrrolidin
1-[2-(N-4-Chlor-3-nitrophenylacetyl-N-methyl-amino)-2-phenylethyl]-3-hydroxy-pyrrolidin
1-[2-(N-5-Chlor-3-nitrophenylacetyl-N-methyl-amino)-2-phenylethyl]-3-hydroxy-pyrrolidin
1-[2-(N-6-Chlor-3-nitrophenylacetyl-N-methyl-amino)-2-phenylethyl]-3-hydroxy-pyrrolidin;
mit 2- oder 3-Chlor-4-nitrophenylacetylchlorid:
1-[2-(N-2-Chlor-4-nitrophenylacetyl-N-methyl-amino)-2-phenylethyl]-3-hydroxy-pyrrolidin
1-[2-(N-3-Chlor-4-nitrophenylacetyl-N-methyl-amino)-2-phenylethyl]-3-hydroxy-pyrrolidin;

mit 3-, 4-, 5- oder 6-Brom-2-nitrophenylacetylchlorid:
1-[2-(N-3-Brom-2-nitrophenylacetyl-N-methyl-amino)-2-phenylethyl]-3-hydroxy-pyrrolidin
1-[2-(N-4-Brom-2-nitrophenylacetyl-N-methyl-amino)-2-phenylethyl]-3-hydroxy-pyrrolidin
1-[2-(N-5-Brom-2-nitrophenylacetyl-N-methyl-amino)-2-phenylethyl]-3-hydroxy-pyrrolidin
1-[2-(N-6-Brom-2-nitrophenylacetyl-N-methyl-amino)-2-phenylethyl]-3-hydroxy-pyrrolidin;
mit 2-, 4-, 5- oder 6-Brom-3-nitrophenylacetylchlorid:
1-[2-(N-2-Brom-3-nitrophenylacetyl-N-methyl-amino)-2-phenylethyl]-3-hydroxy-pyrrolidin
1-[2-(N-4-Brom-3-nitrophenylacetyl-N-methyl-amino)-2-phenylethyl]-3-hydroxy-pyrrolidin
1-[2-(N-5-Brom-3-nitrophenylacetyl-N-methyl-amino)-2-phenylethyl]-3-hydroxy-pyrrolidin
1-[2-(N-6-Brom-3-nitrophenylacetyl-N-methyl-amino)-2-phenylethyl]-3-hydroxy-pyrrolidin;
mit 2- oder 3-Brom-4-nitrophenylacetylchlorid:
1-[2-(N-2-Brom-4-nitrophenylacetyl-N-methyl-amino)-2-phenylethyl]-3-hydroxy-pyrrolidin
1-[2-(N-3-Brom-4-nitrophenylacetyl-N-methyl-amino)-2-phenylethyl]-3-hydroxy-pyrrolidin.

Beispiel 5

Man löst 6 g 3-Chlor-4-hydroxy-phenylessigsäurehydrazid (erhältlich aus 3-Chlor-4-hydroxy-phenylessigsäureethylester mit Hydrazin) in 200 ml Wasser und 40 ml 1 n Salzsäure, tropft unter Rühren bei 0-3° eine Lösung von 2,4 g $NaNO_2$ in 40 ml Wasser hinzu, rührt noch 30 Min. und extrahiert das gebildete Azid mit Dichlormethan. Nach Trocknen über $MgSO_4$ und Konzentrieren auf 50 ml wird die Lösung unter Rühren zugetropft zu einer Lösung von 6,6 g 1-(2-Methylamino-2-phenyl-ethyl)-3-hydroxy-pyrrolidin und 4,4 ml Triethylamin in 100 ml Dichlormethan. Man rührt noch 2 Std. bei 20°, arbeitet wie üblich auf und erhält 1-[2-(N-3-Chlor-4-hydroxy-phenylacetyl-N-methyl-amino)-2-phenyl-ethyl]-3-hydroxy-pyrrolidin.

Beispiel 6

Man hydriert eine Lösung von 1 g 1-[2-(N-p-Benzyloxyphenylacetyl-N-methyl-amino)-2-phenyl-ethyl]-3-hydroxy-pyrrolidin [erhältlich aus 1-(2-Methylamino-2-phenyl-ethyl)-3-hydroxypyrrolidin und p-Benzyloxyphenylacetylchlorid] in 25 ml Ethylacetat an 0,5 g 5%ig. Pd-C bei 20° und 1 bar bis zum Stillstand der Wasserstoffaufnahme, filtriert, dampft ein und erhält 1-[2-(N-p-Hydroxyphenylacetyl-N-methyl-amino)-2-phenyl-ethyl]-3-hydroxy-pyrrolidin.

Analog erhält man durch Hydrogenolyse der entsprechenden o- oder m-Benzyloxyphenylacetyl-derivate:
1-[2-(N-o-Hydroxyphenylacetyl-N-methyl-amino)-2-phenylethyl]-3-hydroxy-pyrrolidin
1-[2-(N-m-Hydroxyphenylacetyl-N-methyl-amino)-2-phenylethyl]-3-hydroxy-pyrrolidin.

Analog erhält man durch Hydrogenolyse von 1-[2-(N-MethylN-m-trifluormethyl-phenylacetyl-amino)-2-o-, -m- oder -p-benzyloxyphenyl-ethyl]-3-hydroxy-pyrrolidin:
1-[2-(N-Methyl-N-m-trifluormethyl-phenylacetyl-amino)-2-o-hydroxyphenyl-ethyl]-3-hydroxy-pyrrolidin
1-[2-(N-Methyl-N-m-trifluormethyl-phenylacetyl-amino)-2-m-hydroxyphenyl-ethyl]-3-hydroxy-pyrrolidin
1-[2-(N-Methyl-N-m-trifluormethyl-phenylacetyl-amino)-2-p-hydroxyphenyl-ethyl]-3-hydroxy-pyrrolidin.

Beispiel 7

Man hydriert eine Lösung von 10 g 1-[2-(N-3-(Benzyloxycarbonyl-amino)-4-pyridyl-acetyl-N-methyl-amino)-2-phenylethyl]-3-hydroxy-pyrrolidin [erhältlich aus 1-(2-Methylamino-2-phenyl-ethyl)-3-hydroxy-pyrrolidin und 3-Benzyloxycarbonylamino-4-pyridyl-acetylchlorid] in 250 ml Methanol an 0,5 g 5%ig. Pd-C bei 20° und 1 bar bis zum Stillstand der Wasserstoffaufnahme, filtriert, dampft ein und erhält 1-[2-(N-(3-Amino-4-pyridyl-acetyl)-N-methyl-amino)-2-phenylethyl]-3-hydroxy-pyrrolidin.

Analog erhält man durch Hydrogenolyse der entsprechenden Benzyloxycarbonyl-derivate:
1-[2-(N-(3-Amino-4-methyl-2-pyridyl-acetyl)-N-methyl-amino)-2-phenyl-ethyl]-3-hydroxy-pyrrolidin
1-[2-(N-(5-Amino-4-methyl-2-pyridyl-acetyl)-N-methyl-amino)-2-phenyl-ethyl]-3-hydroxy-pyrrolidin
1-[2-(N-Methyl-N-m-trifluormethylphenylacetyl-amino)-2-o-aminophenyl-ethyl]-3-hydroxy-pyrrolidin
1-[2-(N-Methyl-N-m-trifluormethylphenylacetyl-amino)-2-m-aminophenyl-ethyl]-3-hydroxy-pyrrolidin
1-[2-(N-Methyl-N-m-trifluormethylphenylacetyl-amino)-2-p-aminophenyl-ethyl]-3-hydroxy-pyrrolidin.

Beispiel 8

Ein Gemisch von 3,82 g 1-[2-(N-p-Carboxyphenylacetyl-N-methyl-amino)-2-phenyl-ethyl]-3-hydroxy-pyrrolidin [erhältlich durch Reaktion von 1-(2-Methylamino-2-phenylethyl)-3-hydroxy-pyrrolidin mit 4-Methoxycarbonyl-phenylacetylchlorid zu 1-[2-(N-p-Methoxycarbonyl-phenylacetyl-N-methyl-amino)-2-phenyl-ethyl]-3-hydroxy-pyrrolidin und anschließende Verseifung], 1,62 g Carbonyldiimidazol und 140 ml DMF wird 1 Std. bei 20° gerührt. Man gibt 15 ml wässerige $NH_3$-Lösung hinzu, rührt weitere 12 Std., dampft ein und nimmt in 1 n wässeriger Salzsäure auf. Man wäscht mit Ethylacetat, arbeitet mit Natronlauge/Ethylacetat wie üblich auf und erhält 1-[2-(N-p-Carbamoyl-phenylacetyl-N-methylamino)-2-phenyl-ethyl]-3-hydroxy-pyrrolidin, Rf 0,12.

Beispiel 9

Eine Lösung von 10 g 1-[2-(N-Methyl-N-p-nitrophenylacetyl-amino)-2-phenyl-ethyl]-3-hydroxy-pyrrolidin (Beispiel 3) in 200 ml Methanol wird bei 20° und Normaldruck an 5 g Raney-Ni hydriert, bis die berechnete Menge aufgenommen ist. Man filtriert, dampft das Filtrat ein und erhält 1-[2-(N-p-Aminophenylacetyl-N-methyl-amino)-2-phenyl-ethyl]-3-hydroxypyrrolidin ("A"), Rf 0,31.

Analog erhält man durch Reduktion der entsprechenden Nitroverbindungen die nachstehenden Verbindungen:

1-[2-(N-p-Aminophenylacetyl-N-methyl-amino)-2-phenylethyl]-3R-hydroxy-pyrrolidin, Rf 0,31; [α] -2,9°; Di-HCl'-monohydrat, F. 61°
1-[2-(N-p-Aminophenylacetyl-N-methyl-amino)-2-phenylethyl]-3S-hydroxy-pyrrolidin, Rf 0,31; [α] -2,9°
1-[2S-(N-p-Aminophenylacetyl-N-methyl-amino)-2-phenylethyl]-3-hydroxy-pyrrolidin, Rf 0,31; [α] +121,5°; Di-HCl'-dihydrat, F. 232°
1-[2-(N-o-Aminophenylacetyl-N-methyl-amino)-2-phenylethyl]-3-hydroxy-pyrrolidin
1-[2-(N-o-Aminophenylacetyl-N-methyl-amino)-2-phenylethyl]-3R-hydroxy-pyrrolidin
1-[2-(N-o-Aminophenylacetyl-N-methyl-amino)-2-phenylethyl]-3S-hydroxy-pyrrolidin, Rf 0,36
1-[2S-(N-o-Aminophenylacetyl-N-methyl-amino)-2-phenylethyl]-3-hydroxy-pyrrolidin, Rf 0,36
1-[2S-(N-o-Aminophenylacetyl-N-methyl-amino)-2-phenylethyl]-3S-hydroxy-pyrrolidin, Rf 0,36; [α] +136,1°; Di-HCl'-dihydrat, F. 206°
1-[2S-(N-o-Aminophenylacetyl-N-methyl-amino)-2-phenylethyl]-3R-hydroxy-pyrrolidin, Rf 0,36
1-[2-(N-m-Aminophenylacetyl-N-methyl-amino)-2-phenylethyl]-3-hydroxy-pyrrolidin
1-[2-(N-m-Aminophenylacetyl-N-methyl-amino)-2-phenylethyl]-3R-hydroxy-pyrrolidin
1-[2-(N-m-Aminophenylacetyl-N-methyl-amino)-2-phenylethyl]-3S-hydroxy-pyrrolidin
1-[2S-(N-m-Aminophenylacetyl-N-methyl-amino)-2-phenylethyl]-3-hydroxy-pyrrolidin, Rf 0,16
1-[2-(N-5-Amino-2-thienyl-acetyl-N-methyl-amino)-2-phenylethyl]-3-hydroxy-pyrrolidin
1-[2-(N-2-Amino-3-chlorphenylacetyl-N-methyl-amino)-2-phenylethyl]-3-hydroxy-pyrrolidin
1-[2-(N-2-Amino-4-chlorphenylacetyl-N-methyl-amino)-2-phenylethyl]-3-hydroxy-pyrrolidin
1-[2-(N-2-Amino-5-chlorphenylacetyl-N-methyl-amino)-2-phenylethyl]-3-hydroxy-pyrrolidin
1-[2-(N-2-Amino-6-chlorphenylacetyl-N-methyl-amino)-2-phenylethyl]-3-hydroxy-pyrrolidin
1-[2-(N-3-Amino-2-chlorphenylacetyl-N-methyl-amino)-2-phenylethyl]-3-hydroxy-pyrrolidin
1-[2-(N-3-Amino-4-chlorphenylacetyl-N-methyl-amino)-2-phenylethyl]-3-hydroxy-pyrrolidin
1-[2-(N-3-Amino-5-chlorphenylacetyl-N-methyl-amino)-2-phenylethyl]-3-hydroxy-pyrrolidin
1-[2-(N-3-Amino-6-chlorphenylacetyl-N-methyl-amino)-2-phenylethyl]-3-hydroxy-pyrrolidin
1-[2-(N-4-Amino-2-chlorphenylacetyl-N-methyl-amino)-2-phenylethyl]-3-hydroxy-pyrrolidin
1-[2-(N-4-Amino-3-chlorphenylacetyl-N-methyl-amino)-2-phenylethyl]-3-hydroxy-pyrrolidin
1-[2-(N-2-Amino-3-bromphenylacetyl-N-methyl-amino)-2-phenylethyl]-3-hydroxy-pyrrolidin
1-[2-(N-2-Amino-4-bromphenylacetyl-N-methyl-amino)-2-phenylethyl]-3-hydroxy-pyrrolidin
1-[2-(N-2-Amino-5-bromphenylacetyl-N-methyl-amino)-2-phenylethyl]-3-hydroxy-pyrrolidin
1-[2-(N-2-Amino-6-bromphenylacetyl-N-methyl-amino)-2-phenylethyl]-3-hydroxy-pyrrolidin
1-[2-(N-3-Amino-2-bromphenylacetyl-N-methyl-amino)-2-phenylethyl]-3-hydroxy-pyrrolidin
1-[2-(N-3-Amino-4-bromphenylacetyl-N-methyl-amino)-2-phenylethyl]-3-hydroxy-pyrrolidin
1-[2-(N-3-Amino-5-bromphenylacetyl-N-methyl-amino)-2-phenylethyl]-3-hydroxy-pyrrolidin
1-[2-(N-3-Amino-6-bromphenylacetyl-N-methyl-amino)-2-phenylethyl]-3-hydroxy-pyrrolidin
1-[2-(N-4-Amino-2-bromphenylacetyl-N-methyl-amino)-2-phenylethyl]-3-hydroxy-pyrrolidin
1-[2-(N-4-Amino-3-bromphenylacetyl-N-methyl-amino)-2-phenylethyl]-3-hydroxy-pyrrolidin.

Beispiel 10

Man löst 3,53 g "A" in 175 ml Dichlormethan und tropft unter Rühren eine Lösung von 0,8 g Acetylchlorid in 10 ml Dichlormethan hinzu. Man rührt 10 Minuten, konzentriert die Lösung und filtriert das erhaltene 1-[2-(N-p-Acetamidophenylacetyl-N-methyl-amino)-2-phenyl-ethyl]-3-hydroxy-pyrrolidin ab. Rf 0,19.

Analog erhält man durch Acylierung der entsprechenden primären Aminoverbindungen:

1-[2-(N-p-Acetamidophenylacetyl-N-methyl-amino)-2-phenylethyl]-3R-hydroxy-pyrrolidin, Rf 0,19; [α] -2,4°. HCl'-Dihydrat, F. 125°
1-[2-(N-p-Acetamidophenylacetyl-N-methyl-amino)-2-phenylethyl]-3S-hydroxy-pyrrolidin, Rf 0,19; [α] +2,4°.
1-[2S-(N-p-Acetamidophenylacetyl-N-methyl-amino)-2-phenylethyl]-3-hydroxy-pyrrolidin
1-[2S-(N-p-Acetamidophenylacetyl-N-methyl-amino)-2-phenylethyl]-3S-hydroxy-pyrrolidin, Rf 0,19
1-[2-(N-o-Acetamidophenylacetyl-N-methyl-amino)-2-phenylethyl]-3-hydroxy-pyrrolidin
1-[2-(N-o-Acetamidophenylacetyl-N-methyl-amino)-2-phenylethyl]-3R-hydroxy-pyrrolidin
1-[2-(N-o-Acetamidophenylacetyl-N-methyl-amino)-2-phenylethyl]-3S-hydroxy-pyrrolidin
1-[2S-(N-o-Acetamidophenylacetyl-N-methyl-amino)-2-phenylethyl]-3-hydroxy-pyrrolidin
1-[2S-(N-o-Acetamidophenylacetyl-N-methyl-amino)-2-phenylethyl]-3S-hydroxy-pyrrolidin, Rf 0,34
1-[2-(N-m-Acetamidophenylacetyl-N-methyl-amino)-2-phenylethyl]-3-hydroxy-pyrrolidin
1-[2-(N-m-Acetamidophenylacetyl-N-methyl-amino)-2-phenylethyl]-3R-hydroxy-pyrrolidin
1-[2-(N-m-Acetamidophenylacetyl-N-methyl-amino)-2-phenylethyl]-3S-hydroxy-pyrrolidin
1-[2S-(N-m-Acetamidophenylacetyl-N-methyl-amino)-2-phenylethyl]-3-hydroxy-pyrrolidin
1-[2-(N-5-Acetamido-2-thienyl-acetyl-N-methyl-amino)-2-phenyl-ethyl]-3-hydroxy-pyrrolidin
1-[2-(N-(3-Acetamido-4-pyridylacetyl)-N-methyl-amino)-2-phenyl-ethyl]-3-hydroxy-pyrrolidin
1-[2-(N-(3-Acetamido-4-methyl-2-pyridylacetyl)-N-methylamino)-2-phenyl-ethyl]-3-hydroxy-pyrrolidin
1-[2-(N-(5-Acetamido-4-methyl-2-pyridylacetyl)-N-methylamino)-2-phenyl-ethyl]-3-hydroxy-pyrrolidin
1-[2-(N-Methyl-N-m-trifluormethylphenylacetyl-amino)-2-o-acetamidophenyl-ethyl]-3-hydroxy-pyrrolidin
1-[2-(N-Methyl-N-m-trifluormethylphenylacetyl-amino)-2-m-acetamidophenyl-ethyl]-3-hydroxy-pyrrolidin
1-[2-(N-Methyl-N-m-trifluormethylphenylacetyl-amino)-2-p-acetamidophenyl-ethyl]-3-hydroxy-pyrrolidin
1-[2-(N-Methyl-N-o-methylsulfonylamino-phenylacetyl-amino)-2-phenyl-ethyl]-3-hydroxy-pyrrolidin
1-[2S-(N-Methyl-N-o-methylsulfonylamino-phenylacetyl-amino)-2-phenyl-ethyl]-3S-hydroxy-pyrrolidin, Rf 0,51
1-[2-(N-Methyl-N-m-methylsulfonylamino-phenylacetyl-amino)-2-phenyl-ethyl]-3-hydroxy-pyrrolidin
1-[2-(N-Methyl-N-p-methylsulfonylamino-phenylacetyl-amino)-2-phenyl-ethyl]-3-hydroxy-pyrrolidin
1-[2-(N-Methyl-N-5-methylsulfonylamino-2-thienylacetylamino)-2-phenyl-ethyl]-3-hydroxy-pyrrolidin
1-[2-(N-Methyl-N-(3-methylsulfonylamino-4-pyridylacetyl)-amino)-2-phenyl-ethyl]-3-hydroxy-pyrrolidin
1-[2-(N-Methyl-N-(3-methylsulfonylamino-4-methyl-2-pyridylacetyl)-amino)-2-phenyl-ethyl]-3-hydroxy-pyrrolidin
1-[2-(N-Methyl-N-(5-methylsulfonylamino-4-methyl-2-pyridylacetyl)-amino)-2-phenyl-ethyl]-3-hydroxy-pyrrolidin
1-[2-(N-Methyl-N-m-trifluormethylphenylacetyl-amino)-2-o-methylsulfonylaminophenyl-ethyl]-3-hydroxy-pyrrolidin
1-[2-(N-Methyl-N-m-trifluormethylphenylacetyl-amino)-2-m-methylsulfonylaminophenyl-ethyl]-3-hydroxy-pyrrolidin
1-[2-(N-Methyl-N-m-trifluormethylphenylacetyl-amino)-2-p-methylsulfonylaminophenyl-ethyl]-3-hydroxy-pyrrolidin.

Beispiel 11

Ein Gemisch von 1 g "A" und 10 ml HCOOH wird 2 Std. gekocht und dann eingedampft. Nach üblicher Aufarbeitung erhält man 1-[2-(N-p-Formamidophenylacetyl-N-methyl-amino)-2-phenylethyl]-3-hydroxy-pyrrolidin.

Analog erhält man durch Formylierung der entsprechenden primären Aminoverbindungen:

1-[2-(N-p-Formamidophenylacetyl-N-methyl-amino)-2-phenylethyl]-3R-hydroxy-pyrrolidin
1-[2-(N-p-Formamidophenylacetyl-N-methyl-amino)-2-phenylethyl]-3S-hydroxy-pyrrolidin
1-[2S-(N-p-Formamidophenylacetyl-N-methyl-amino)-2-phenylethyl]-3-hydroxy-pyrrolidin
1-[2-(N-o-Formamidophenylacetyl-N-methyl-amino)-2-phenylethyl]-3-hydroxy-pyrrolidin
1-[2-(N-o-Formamidophenylacetyl-N-methyl-amino)-2-phenylethyl]-3R-hydroxy-pyrrolidin
1-[2-(N-o-Formamidophenylacetyl-N-methyl-amino)-2-phenylethyl]-3S-hydroxy-pyrrolidin

1-[2S-(N-o-Formamidophenylacetyl-N-methyl-amino)-2-phenylethyl]-3S-hydroxy-pyrrolidin
1-[2-(N-m-Formamidophenylacetyl-N-methyl-amino)-2-phenylethyl]-3-hydroxy-pyrrolidin
1-[2-(N-m-Formamidophenylacetyl-N-methyl-amino)-2-phenylethyl]-3R-hydroxy-pyrrolidin
1-[2-(N-m-Formamidophenylacetyl-N-methyl-amino)-2-phenylethyl]-3S-hydroxy-pyrrolidin
1-[2S-(N-m-Formamidophenylacetyl-N-methyl-amino)-2-phenylethyl]-3S-hydroxy-pyrrolidin
1-[2-(N-5-Formamido-2-phenylacetyl-N-methyl-amino)-2-phenylethyl]-3-hydroxy-pyrrolidin
1-[2-(N-3-Formamido-4-pyridylacetyl-N-methyl-amino)-2-phenylethyl]-3-hydroxy-pyrrolidin
1-[2-(N-3-Formamido-4-methyl-2-pyridylacetyl-N-amino)-2-phenyl-ethyl]-3-hydroxy-pyrrolidin
1-[2-(N-5-Formamido-4-methyl-2-pyridylacetyl-N-amino)-2-phenyl-ethyl]-3-hydroxy-pyrrolidin
1-[2-(N-Methyl-N-m-trifluormethylphenylacetyl-amino)-2-o-formamidophenyl-ethyl]-3-hydroxy-pyrrolidin
1-[2-(N-Methyl-N-m-trifluormethylphenylacetyl-amino)-2-m-formamidophenyl-ethyl]-3-hydroxy-pyrrolidin
1-[2-(N-Methyl-N-m-trifluormethylphenylacetyl-amino)-2-p-formamidophenyl-ethyl]-3-hydroxy-pyrrolidin

Beispiel 12

Man löst 4,26 g "A"-Dihydrochlorid in 50 ml Wasser und versetzt mit 0,81 g KCNO. Nach 3 Std. Rühren bei 20° engt man ein und erhält 1-[2-(N-Methyl-N-p-ureidophenylacetylamino)-2-phenyl-ethyl]-3-hydroxy-pyrrolidin, Rf 0,09.

Analog erhält man aus den entsprechenden primären Aminen:
1-[2-(N-Methyl-N-p-ureidophenylacetyl-amino)-2-phenylethyl]-3R-hydroxy-pyrrolidin, Rf 0,09; [α] -2,7°. HCl'-Trihydrat, F. 94°.
1-[2-(N-Methyl-N-p-ureidophenylacetyl-amino)-2-phenylethyl]-3S-hydroxy-pyrrolidin, Rf 0,09; [α] +2,7°.
1-[2S-(N-Methyl-N-p-ureidophenylacetyl-amino)-2-phenylethyl]-3-hydroxy-pyrrolidin, Rf 0,09; [α] +114,0°.
1-[2-(N-Methyl-N-o-ureidophenylacetyl-amino)-2-phenylethyl]-3-hydroxy-pyrrolidin
1-[2-(N-Methyl-N-o-ureidophenylacetyl-amino)-2-phenylethyl]-3R-hydroxy-pyrrolidin
1-[2-(N-Methyl-N-o-ureidophenylacetyl-amino)-2-phenylethyl]-3S-hydroxy-pyrrolidin
1-[2S-(N-Methyl-N-o-ureidophenylacetyl-amino)-2-phenylethyl]-3-hydroxy-pyrrolidin, Rf 0,20; [α] +107,9°. HCl'-Trihydrat, F. 151°
1-[2S-(N-Methyl-N-o-ureidophenylacetyl-amino)-2-phenylethyl]-3S-hydroxy-pyrrolidin, Rf 0,20.
1-[2-(N-Methyl-N-m-ureidophenylacetyl-amino)-2-phenylethyl]-3-hydroxy-pyrrolidin
1-[2-(N-Methyl-N-m-ureidophenylacetyl-amino)-2-phenylethyl]-3R-hydroxy-pyrrolidin
1-[2-(N-Methyl-N-m-ureidophenylacetyl-amino)-2-phenylethyl]-3S-hydroxy-pyrrolidin
1-[2S-(N-Methyl-N-m-ureidophenylacetyl-amino)-2-phenylethyl]-3-hydroxy-pyrrolidin, Rf 0,11
1-[2-(N-Methyl-N-5-ureido-2-thienylacetyl-amino)-2-phenylethyl]-3-hydroxy-pyrrolidin
1-[2-(N-Methyl-N-3-ureido-4-pyridylacetyl-amino)-2-phenylethyl]-3-hydroxy-pyrrolidin
1-[2-(N-Methyl-N-3-ureido-4-methyl-pyridylacetyl-amino)-2-phenyl-ethyl]-3-hydroxy-pyrrolidin
1-[2-(N-Methyl-N-5-ureido-4-methyl-pyridylacetyl-amino)-2-phenyl-ethyl]-3-hydroxy-pyrrolidin
1-[2-(N-Methyl-N-m-trifluormethylphenylacetyl-amino)-2-o-ureidophenyl-ethyl]-3-hydroxy-pyrrolidin
1-[2-(N-Methyl-N-m-trifluormethylphenylacetyl-amino)-2-m-ureidophenyl-ethyl]-3-hydroxy-pyrrolidin
1-[2-(N-Methyl-N-m-trifluormethylphenylacetyl-amino)-2-p-ureidophenyl-ethyl]-3-hydroxy-pyrrolidin.

Beispiel 13

Ein Gemisch von 3,53 g "A", 0,57 g Methylisocyanat und 50 ml THF wird 30 Min. bei 20° gerührt. Man dampft ein, arbeitet wie üblich auf und erhält 1-[2-(N-Methyl-N-p-(N'-methylureido)-phenylacetyl-amino)-2-phenyl-ethyl]-3-hydroxypyrrolidin.

Analog erhält man aus den entsprechenden primären Aminen:
1-[2-(N-Methyl-N-o-(N'-methyl-ureido)-phenylacetyl-amino)-2-phenyl-ethyl]-3-hydroxy-pyrrolidin
1-[2-(N-Methyl-N-m-(N'-methyl-ureido)-phenylacetyl-amino)-2-phenyl-ethyl]-3-hydroxy-pyrrolidin
1-[2-(N-Methyl-N-3-(N'-methylureido)-4-pyridylacetylamino)-2-phenyl-ethyl]-3-hydroxy-pyrrolidin
1-[2-(N-Methyl-N-3-(N'-methylureido)-4-methyl-2-pyridylacetyl-amino)-2-phenyl-ethyl]-3-hydroxy-pyrrolidin
1-[2-(N-Methyl-N-5-(N'-methylureido)-4-methyl-2-pyridylacetyl-amino)-2-phenyl-ethyl]-3-hydroxy-pyrrolidin.

Beispiel 14

a) Analog Beispiel 1 erhält man aus 1-(2-Methylamino-2S-phenyl-ethyl)-3S-hydroxy-pyrrolidin und 4-Methoxy-3-nitro-phenylacetylchlorid das 1-[2-(N-(4-Methoxy-3-nitro-phenylacetyl)-N-methyl-amino)-2S-phenyl-ethyl]-3S-hydroxy-pyrrolidin; Rf 0,44.

b) Durch Hydrierung analog Beispiel 9 erhält man aus der nach a) hergestellten Verbindung das 1-[2-(N-(3-Amino-4-methoxy-phenylacetyl)-N-methyl-amino)-2S-phenyl-ethyl]-3S-hydroxy-pyrrolidin; Rf 0,31.

Die nachstehenden Beispiele betreffen pharmazeutische Zubereitungen, die Amine der Formel I oder ihre Säureadditionssalze enthalten:

Beispiel A: Tabletten

Ein Gemisch von 0,1 kg 1-[2S-(N-o-Aminophenylacetyl-N-methylamino)-2-phenyl-ethyl]-3-hydroxy-pyrrolidin-dihydrochloriddihydrat, 4 kg Lactose, 1,2 kg Kartoffelstärke, 0,2 kg Talk und 0,1 kg Magnesiumstearat wird in üblicher Weise zu Tabletten gepreßt, derart, daß jede Tablette 10 mg Wirkstoff enthält.

Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C: Kapseln

2 kg 1-[2S-(N-Methyl-N-o-ureidophenylacetyl-amino)-2-phenylethyl]-3-hydroxy-pyrrolidin-hydrochlorid-dihydrat werden in üblicher Weise in Hartgelatinekapseln gefüllt, so daß jede Kapsel 20 mg des Wirkstoffs enthält.

Beispiel D: Ampullen

Eine Lösung von 1-[2S-(N-p-Aminophenylacetyl-N-methylamino)-2-phenyl-ethyl]-3-hydroxy-pyrrolidin-dihydrochloriddihydrat in 15 1 1,2-Propandiol und 15 1 zweifach destilliertem Wasser wird steril filtriert, in Ampullen abgefüllt, und steril verschlossen. Jede Ampulle enthält 2 mg Wirkstoff.

Analog sind Tabletten, Dragees, Kapseln und Ampullen erhältlich, die einen oder mehrere der übrigen Wirkstoffe der Formel I und/oder ihrer physiologisch unbedenklichen Salze enthalten.

**Patentansprüche**

**1.** 1-(2-Arylethyl)-pyrrolidine der Formel I

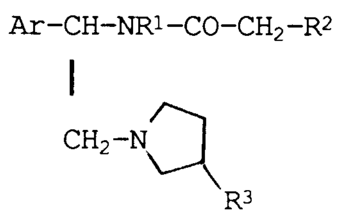

I

worin

Ar eine unsubstituierte oder eine einfach durch OH, $-O-CO-NH_2$, -O-CO-NHA, $-O-CO-NA_2$, $NH_2$, -NH-CHO, -NH-CO-A, $-NH-CO-NH_2$, -NH-CO-NHA, oder $NH-SO_2-A$ substituierte Phenylgruppe,

$R^1$ A,

$R^2$ eine unsubstituierte oder eine ein- oder zweifach durch A, Hal, $CF_3$, OH, OA, $-O-CO-NH_2$, -O-CO-NHA, $-O-CO-NA_2$, $NO_2$, $NH_2$, -NH-CHO, -NH-CO-A, $-NH-CO-NH_2$, -NH-CO-NHA, $-NH-SO_2A$, -CO-A, $-CONH_2$, -CONHA, $-CONA_2$, $-CH_2-CONH_2$ und/oder $-O-CH_2-CONH_2$ substituierte Phenyl-, Naphthyl-, Thienyl-, Benzothienyl- oder Pyridylgruppe,

$R^3$ OH oder $CH_2OH$,

A Alkyl mit 1-4 C-Atomen und

Hal F, Cl, Br oder I bedeuten,

sowie deren Salze.

**2.**

a) 1-[2-(N-3,4-Dichlorphenylacetyl-N-methyl-amino)-2-phenyl-ethyl]-3-hydroxy-pyrrolidin;

b) 1-[2-(N-3,4-Dichlorphenylacetyl-N-methyl-amino)-2-phenyl-ethyl]-3-hydroxymethyl-pyrrolidin;
c) 1-[2-(N-Methyl-N-p-nitrophenylacetyl-amino)-2-phenyl-ethyl]-3-hydroxy-pyrolidin;
d) 1-[2-(N-p-Aminophenylacetyl-N-methyl-amino)-2-phenyl-ethyl]-3-hydroxy-pyrrolidin;
e) 1-[2-(N-p-Acetamidophenylacetyl-N-methyl-amino)-2-phenyl-ethyl]-3-hydroxy-pyrrolidin;
f) 1-[2-(N-Methyl-N-p-ureidophenylacetyl-amino)-2-phenyl-ethyl]-3-hydroxy-pyrrolidin;
g) 1-[2-(N-o-Aminophenylacetyl-N-methyl-amino)-2-phenyl-ethyl]-3-hydroxy-pyrrolidin;
h) 1-[2-(N-Methyl-N-o-ureidophenylacetyl-amino)-2-phenyl-ethyl]-3-hydroxy-pyrrolidin;
i) 1-[2-(N-m-Aminophenylacetyl-N-methyl-amino)-2-phenyl-ethyl]-3-hydroxy-pyrrolidin;
sowie deren Salze.

**3.** Verfahren zur Herstellung von 1-(2-Phenylethyl)-pyrrolidinen der Formel I gemäß Anspruch 1 sowie ihrer Salze, dadurch gekennzeichnet, daß man eine Verbindung der Formel II

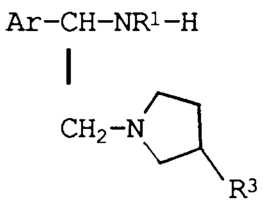

II

worin Ar, $R^1$ und $R^3$ die bei Formel I angegebene Bedeutung haben, mit einer Verbindung der Formel III

$HOOC\text{-}CH_2\text{-}R^2$ III

worin $R^2$ die bei Formel I angegebene Bedeutung hat, oder einem ihrer funktionellen Derivate umsetzt

oder daß man eine Verbindung, die sonst der Formel I entspricht, aber an Stelle eines oder mehrerer H-Atome eine oder mehrere reduzierbare oder hydrogenolytisch abspaltbare Gruppen und/oder C-C- und/oder C-N-Bindungen enthält, mit einem reduzierenden Mittel behandelt,

oder daß man zur Herstellung einer Verbindung der Formel I, die eine Säureamidgruppe enthält, eine entsprechende Carbonsäure oder einen ihrer Ester mit Ammoniak oder mit einem Amin der Formeln $A\text{-}NH_2$ oder $A_2HH$ umsetzt,

und/oder daß man in einer Verbindung der Formel I einen oder mehrere der Reste Ar und/oder $R^2$ in einen oder mehrere andere Reste Ar und/oder $R^2$ umwandelt,

und/oder daß man eine Base der Formel I durch Behandeln mit einer Säure in eines ihrer Salze überführt.